Europäisches Patentamt

European Patent Office

Office européen des brevets

(11) Veröffentlichungsnummer: **0 275 551**
**A1**

## (12) EUROPÄISCHE PATENTANMELDUNG

(21) Anmeldenummer: 87119293.6

(22) Anmeldetag: 29.12.87

(51) Int. Cl.⁴: **C07D 223/10**

(30) Priorität: 09.01.87 DE 3700451

(43) Veröffentlichungstag der Anmeldung:
27.07.88 Patentblatt 88/30

(84) Benannte Vertragsstaaten:
CH DE FR GB IT LI

(71) Anmelder: **BAYER AG**

**D-5090 Leverkusen 1, Bayerwerk(DE)**

(72) Erfinder: **Blank, Heinz-Ulrich, Dr.**
**Am Geusfelde 35**
**D-5068 Odenthal-Glöbusch(DE)**
Erfinder: **Bauer, Wolfgang, Dr.**
**Karl-Krekeler-Strasse 17**
**D-5090 Leverkusen(DE)**

(54) **Verfahren zur Herstellung von N-Methylol-caprolactam.**

(57) Das Verfahren zur Herstellung von N-Methylol-caprolactam durch Umsetzung von Caprolactam mit Formaldehyd in Gegenwart eines alkalischen Katalysators bei erhöhter Temperatur wird wesentlich verbessert, wenn man die Umsetzung weitgehend lösungsmittelfrei bei 70 bis 100°C durchführt. In vorteilhafter Weise liegt der verwendete alkalische Katalysator in heterogener Form vor. In weiterhin vorteilhafter Weise wird der Reaktionsansatz nach dem Abkühlen auf eine Temperatur unterhalb des Schmelzpunktes von reinem N-Methylol-caprolactam mit Impfkristallen aus reinem N-Methylol-caprolactam versetzt.

EP 0 275 551 A1

0 275 551

## Verfahren zur Herstellung von N-Methylol-caprolactam

Die vorliegende Erfindung betrifft ein verbessertes Verfahren zur Herstellung von N-Methylol-caprolactam durch Umsetzung von Caprolactam mit Formaldehyd in Gegenwart eines alkalischen Katalysators bei erhöhter Temperatur.

Es ist bereits bekannt, aus Caprolactam und überschüssigem Paraformaldehyd (50 % Überschuß) in 95 %igem Ethanol in Gegenwart von 4,2 Mol-% Natriumhydroxid als Katalysator N-Methylol-caprolactam mit einer Ausbeute von 67 % der theoretischen Ausbeute herzustellen (J. Am. Chem. Soc. 70 (1948), 2115-2118). Die angegebene Umsetzung wird in etwa 50 %iger Lösung durchgeführt.

Die genannte Vorschrift zur Herstellung von N-Methylol-caprolactam findet sich auch in Houben-Weyl, Methoden der organischen Chemie, 4. Aufl., Bd. XI/2 (1958) S. 570, worin jedoch angegeben wird, daß die zitierte Vorschrift nicht reproduziert werden konnte und eine Ausbeute von nur 50 % der theoretischen Ausbeute erreicht wurde. Eine sehr ähnliche Vorschrift findet sich ferner in DE-OS 2 616 374 mit ebenfalls nur 50 % der theoretischen Ausbeute. Ein kurzer Hinweis auf die Herstellung von N-Methylol-caprolactam als bloße Zwischenstufe zur Herstellung weiterer Caprolactamderivate findet sich ferner in Arch. Pharm. 294 (1961), S. 344-348). In diesem Hinweis sind jedoch nur die Ausgangsprodukte Caprolactam und Paraformaldehyd genannt; es fehlen weitere Hinweise zu den Reaktionsparametern. Das N-Methylol-caprolactam wird hierin als Öl erhalten; jede Angabe zur Charakterisierung und zur Ausbeute des hergestellten N-Methylol-caprolactams fehlt.

In US 3.073.843 findet sich ferner eine Herstellungsvorschrift für eine formal ähnliche Verbindung, nämlich für N-Methylol-pyrrolidon, worin zu vorgelegtem Pyrrolidon Kaliumhydroxid gegeben wurde, woraufhin eine Suspension von Kalium-pyrrolidon im restlichen Pyrrolidon gebildet wird. Daraufhin wird Paraformaldehyd zugesetzt, was zu einer spontanen Temperaturerhöhung auf 80°C führt. Die Ausbeute wird als im wesentlichen quantitativ angegeben. Dieses Verfahren ist jedoch nicht auf Caprolactam zu übertragen, weil

a) 2-Pyrrolidon im Gegensatz zu Caprolactam bei nur wenig erhöhter Temperatur schon flüssig ist;

b) das 5-Ringsystem des 2-Pyrrolidons und das 7-Ringsystem des Caprolactams unterschiedlich reaktiv sind. So erwärmt sich die Reaktionsmischung gemäß US 3.073.843 spontan auf etwa 80°C, während das Gemisch aus Caprolactam, Paraformaldehyd und Kaliumhydroxid durch Energiezufuhr zunächst aufgeschmolzen werden muß und dann unter weiterer Energiezufuhr auf eine geeignete Reaktionstemperatur gebracht werden und dort gehalten werden muß;

c) das N-Methylol-caprolactam thermisch labil ist, während im Gegensatz hierzu das N-Methylol-pyrrolidon eine stabile Verbindung ist.

Es wurde nun überraschenderweise gefunden, daß N-Methylol-caprolactam in Ausbeuten hergestellt werden kann, die weit über denen der Literatur liegen, wenn man die Umsetzung weitgehend lösungsmittelfrei durchführt.

Es wurde ein Verfahren zur Herstellung von N-Methylol-caprolactam (N-Hydroxymethyl caprolactam) durch Umsetzung von Caprolactam mit Formaldehyd in Gegenwart eines alkalischen Katalysators bei erhöhter Temperatur gefunden, das dadurch gekennzeichnet ist, daß die Umsetzung weitgehend lösungsmittelfrei bei einer Temperatur von 70 bis 100°C durchgeführt wird.

Das erfindungsgemäße Verfahren wird weitgehend lösungsmittelfrei durchgeführt. Weitgehend lösungsmittelfrei bedeutet hierbei den Einsatz von OH-Gruppen enthaltenden Lösungsmitteln, wie $H_2O$ oder $C_1-C_4$-Alkanolen (Methanol, Ethanol, Propanol, Isopropanol, Butanol, Isobutanol; bevorzugt Methanol, Ethanol), bis zu höchstens 10 Gew.-%, beispielsweise 0,01 bis 10 Gew.-%, bevorzugt 0,01 bis 5 Gew.-%, bezogen auf den Gesamtreaktionsansatz, den Einsatz von aprotischen Lösungsmitteln, wie aliphatischen oder aromatischen Kohlenwasserstoffen oder aliphatischen oder aromatischen Halogenkohlenwasserstoffen (Hexan, Heptan, Oktan, Isooktan, Dodecan, Isododecan, Benzol, Toluol, Xylol, Chlorbenzol, Dichlorbenzol, Methylenchlorid, Chloroform, Tetrachlorkohlenstoff, halogenierte Ethane oder Propane und ähnliche), bis zu höchstens 20 Gew.-%, beispielsweise 0,01 bis 20 Gew.-%, bevorzugt 0,01 bis 10 Gew.-%, bezogen auf dem Gesamtreaktionsansatz, oder den völligen Verzicht auf Lösungs-oder Verdünnungsmittel. Für den Fall, daß ein Lösungs-oder Verdünnungsmittel eingesetzt werden soll, kann auch ein Gemisch der genannten Lösungsmittel eingesetzt werden. Bevorzugt wird ein aprotisches Lösungsmittel(gemisch) eingesetzt oder ohne Lösungs-oder Verdünnungsmittel gearbeitet. Die Arbeitsweise ohne jedes Lösungs-oder Verdünnungsmittel ist besonders bevorzugt.

Als alkalischer Katalysator eignen sich sowohl alkalisch reagierende anorganische Verbindungen, wie die Hydroxide von Alkalimetallen oder Erdalkalimetallen sowie die Salze schwacher anorganischer Säuren der Alkalimetalle, als auch alkalisch reagierende organische Verbindungen, wie tertiäre aliphatische Amine, beispielsweise Triethylamin. In bevorzugter Weise werden alkalische Katalysatoren benutzt, die in fester

2

Form in das Reaktionsgemisch eingebracht werden können und während der Reaktion heterogen vorliegen. Dies sind beispielsweise die genannten Hydroxide von Alkalimetallen oder Erdalkalimetallen sowie die Salze schwacher anorganischer Säuren von Alkalimetallen. Solche Salze schwacher anorganischer Säuren sind beispielsweise Natriumtetraborat (Borax), Natriumcarbonat (Soda) und Kaliumcarbonat (Pottasche). Von diesen werden bevorzugt die Carbonate, besonders bevorzugt das Kaliumcarbonat, eingesetzt.

Der alkalische Katalysator wird in Mengen von 0,1 bis 2,5 Mol-%, bevorzugt 0,25 bis 1,5 Mol-%, besonders bevorzugt 0,5 bis 1,0 Mol-%, bezogen auf das Caprolactam, eingesetzt.

Das erfindungsgemäße Verfahren wird bei einer Temperatur von 70 bis 100°C, bevorzugt 70 bis 90°C, besonders bevorzugt 70 bis 80°C, durchgeführt. Das erfindungsgemäße Verfahren ergibt in dieser Form Ausbeuten von 80 % und mehr der theoretischen Ausbeute.

Es wurde ferner gefunden, daß man die Ausbeute noch weiter bis auf über 95 % der theoretischen Ausbeute steigern kann, wenn man dem Reaktionsansatz nach dem Abkühlen auf eine Temperatur unterhalb des Schmelzpunktes von reinem N-Methylol-caprolactam Impfkristalle aus reinem N-Methylol-caprolactam zusetzt. Als Schmelzpunkt des N-Methylol-caprolactams wird hierbei von einer Temperatur von 65 bis 66°C ausgegangen. Es ist sehr überraschend, daß das beschriebene Animpfen nicht nur eine - schnellere Gewinnung des N-Methylol-caprolactams in kristalliner Form ermöglicht, sondern daß eine höhere Umsetzung und damit Ausbeute erzielt werden, ohne daß der Mechanismus dieses Vorgangs bisher aufgeklärt werden konnte. Das beschriebene Animpfen stellt daher eine bevorzugte Ausführungsvariante des erfindungsgemäßen Verfahrens dar.

Das Animpfen wird bei einer Temperatur unterhalb des Schmelzpunktes von reinem N-Methylol-caprolactam, bevorzugt bei 10 bis 65°C, besonders bevorzugt bei 20 bis 60°C und ganz besonders bevorzugt bei 30 bis 55°C, durchgeführt. Die Impfkristalle werden dem in der genannten Weise abgekühlten Reaktionsansatz in einer Menge von 0,0001 bis 10 Gew.-%, bevorzugt 0,001 bis 5 Gew.-%, besonders bevorzugt 0,1 bis 1 Gew.-%, bezogen auf den Gesamtreaktionsansatz, zugegeben.

Das erfindungsgemäße Verfahren kann beispielsweise so durchgeführt werden, daß man Caprolactam, Formaldehyd und den Katalysator vorvermischt und diese Mischung portionsweise in ein vorgeheiztes Reaktionsgefäß unter Aufschmelzen einträgt. Ein evtl. mitverwendetes Lösungs-oder Verdünnungsmittel kann vorgelegt oder mit den genannten Stoffen vorvermischt, aber auch separat zugegeben werden. Als Formaldehyd wird eine wasserfreie Form, beispielsweise Paraformaldehyd oder Trioxan, bevorzugt Paraformaldehyd, gewählt. Zur Erzielung hoher Ausbeuten wird ein annähernd äquimolares Gemisch aus Caprolactam und Formaldehyd eingesetzt. Selbstverständlich ist auch ein Abweichen vom äquimolaren Verhältnis von Caprolactam und Formaldehyd möglich, jedoch führt das in bekannter Weise zu einem unvollständigen Umsatz des im Überschuß eingesetzten Stoffes. Die aufgeschmolzene Mischung wird sodann unter Rühren auf die Reaktionstemperatur gebracht.

Nach dem Abkühlen des Reaktionsansatzes auf ein Temperatur unterhalb des Schmelzpunktes von reinem N-Methylol-caprolactam werden Impfkristalle aus reinem N-Methylol-caprolactam in der beschriebenen Weise dem Reaktionsansatz zugesetzt.

Beispiele 1 bis 7

Alle Ansätze wurden in Vierhalskolben, versehen mit Innenthermometer, Rührer und Rückflußkühler, durchgeführt.

Caprolactam, Paraformaldehyd und Katalysator wurden vorvermischt und portionsweise unter Aufschmelzen in den beheizten Kolben eingetragen. Die Reaktionstemperatur lag bei 75°C, die Reaktionszeit betrug mit Ausnahme von Beispiel 7 ≤2 Stunden. Nach der Reaktion und dem Abkühlen wurde in Flaschen abgefüllt. Ausbeute und Umsatz wurden mit Hochdruck-Flüssigkeitschromatographie (HFC) und durch NMR-Spektrum bestimmt.

Beispiel 1

1.130 g (10,00 Mol) 6-Caprolactam, 306,1 g (10,00 Mol) Paraformaldehyd 98 %ig und 6,91 g $K_2CO_3$ - (0,05 Mol), zermahlen, wurden zur Reaktion gebracht. Bis auf den Katalysator lag eine klare Lösung vor. Nach 2 Stunden wurde auf 50°C abgekühlt; einige Krümel reines N-Methylol-caprolactam wurden als Impfkristalle zugefügt. Man erhielt 1.428 g Methylolcaprolactam (= 99,9 % der theoretischen Ausbeute).

## Beispiel 2

113 g (1,00 Mol) 6-Caprolactam, 30,6 g (1,00 Mol) Paraformaldehyd 98 %ig und 3,45 g K₂CO₃ (0,025 Mol), wurden zur Reaktion gebracht. Nach 0,5 Stunden Reaktion bei 75°C wurde die bis auf den Katalysator klare Lösung auf 50°C abgekühlt, dann wurden 0,5 % (bezogen auf Gesamtansatz) Impfkristalle zugesetzt. Man erhielt 147 g kristallisierte Reaktionsmischung (94,4 %ige Ware) = 139 g Methylolcaprolactam = 99.9 % der theoretischen Ausbeute.

## Beispiel 3

Es wurde wie in Beispiel 1 vorgegangen, nur wurde der Katalysator K₂CO₃ als 33 %ige wäßrige Lösung zugesetzt und die Laufzeit auf 0,5 Stunden verkürzt. Man erhielt bei einem 1 Mol-Ansatz 123 g Methylolcaprolactam als Öl = 86 % der theoretischen Ausbeute.

## Beispiel 4

113 g (1,00 Mol) 6-Caprolactam, 30,6 g (1,00 Mol) Paraformaldehyd 98 %ig und 1,38 g K₂CO₃ (0,01 Mol), wurden zur Reaktion (0,5 h, 75°C) gebracht. Danach wurde auf Raumtemperatur ohne Zusetzen von Impfkristallen abgekühlt.
Man erhielt 145 g Reaktionsmischung als Öl (84,8 %ige Ware) = 123 g Methylolcaprolactam = 0,86 Mol = 86 % der theoretischen Ausbeute.

## Beispiel 5 (Vergleichsbeispiel)

Das Vorgehen war wie in Beispiel 2, nur wurden 2,5 Mol-% Essigsäure als Katalysator zugesetzt. Die Ausbeute lag bei 0,5 % der theoretischen Ausbeute.

## Beispiel 6

Ansatzgröße siehe Beispiel 2, anstelle von K₂CO₃ wurden 0,4 Mol-% KOH zugegeben. Die Ausbeute betrug 87 % der theoretischen Ausbeute.

## Beispiel 7

113 g (1,00 Mol) 6-Caprolactam, 30,6 g (1,00 Mol) Paraformaldehyd 98 %ig und 1,6 g (1,6 Mol-%) Triethylamin wurden 15 Stunden bei 85°C gerührt. Die Ausbeute lag bei 80 % der theoretischen Ausbeute.
Die folgende Tabelle zeigt die Ergebnisse nochmals:

| Bsp. | Katalysator | (Menge Mol-%) | Ausbeute | Bemerkung |
|------|-------------|---------------|----------|-----------|
| 1 | $K_2CO_3$ | (0,5) | 99,9 % | |
| 2 | $K_2CO_3$ | (2,5) | 97 % | |
| 3 | $K_2CO_3$ | (0,5) | 86 % | Katalysator als 33 %ige wäßrige Lösung eingesetzt |
| 4 | $K_2CO_3$ | (1,0) | 86 % | ohne Zusatz von Impfkristallen |
| 5 | $CH_3COOH$ | (2,5) | 0,5 % | |
| 6 | KOH | (0,4) | 87 % | |
| 7 | $Et_3N$ | (1,6) | 80 % | |

Beispiel 8

113 g Caprolactam, 30,61 g Paraformaldehyd, 0,69 g $K_2CO_3$ und 7,6 g $H_2O$ (5 Gew.-% auf den Gesamtansatz) wurden nach Beispiel 1 zur Reaktion gebracht. Nach NMR-spektroskopischer Analyse wurde ein 82 %iger Umsatz zum N-Methylol-caprolactam erzielt.

Beispiel 9

113 g Caprolactam, 30,61 g Paraformaldehyd, 0,69 g $K_2CO_3$ und 7,6 g Toluol (5 Gew.-% auf den Gesamtansatz) wurden nach Beispiel 1 zur Reaktion gebracht. Nach NMR-spektroskopischer Analyse wurde ein 91 %iger Umsatz zum N-Methylol-caprolactam erzielt. Nach 24 Std. konnten 70 % der genannten Ausbeute als Kristallisat erhalten werden.

Beispiel 10

113 g Caprolactam, 30,61 g Paraformaldehyd, 0,69 g $K_2CO_3$ und 16 g Heptan (10 Gew.-% auf den Gesamtansatz) wurden nach Beispiel 1 zur Reaktion gebracht. Nach NMR-spektroskopischer Analyse wurde ein 92 %iger Umsatz zum N-Methylol-caprolactam erzielt. Nach 2 Std. konnte die gesamte Ausbeute als Kristallisat erhalten werden.

**Ansprüche**

1. Verfahren zur Herstellung von N-Methylol-caprolactam (N-Hydroxymethyl-caprolactam) durch Umsetzung von Caprolactam mit wasserfreiem Formaldehyd in Gegenwart eines alkalischen Katalysators bei erhöhter Temperatur, dadurch gekennzeichnet, daß die Umsetzung weitgehend lösungsmittelfrei bei einer Temperatur von 70 bis 100°C, bevorzugt von 70 bis 90°C, besonders bevorzugt von 70 bis 80°C, durchgeführt wird.

2. Verfahren nach Anspruch 1, dadurch gekennzeichnet, daß die weitgehend lösungsmittelfreie Durchführung den Einsatz von bis zu 10 Gew.-% eines OH-Gruppen enthaltenden Lösungsmittels oder den Einsatz von bis zu 20 Gew.-% eines aprotischen Lösungsmittels, jeweils bezogen auf den Gesamtreaktionsansatz, oder den Verzicht auf Lösungs-oder Verdünnungsmittel einschließt.

3. Verfahren nach Anspruch 2, dadurch gekennzeichnet, daß bis zu 20 Gew.-% eines aprotischen Lösungsmittels eingesetzt werden oder auf den Einsatz eines Lösungs-oder Verdünnungsmittels verzichtet wird.

4. Verfahren nach Anspruch 3, dadurch gekennzeichnet, daß auf den Einsatz eines Lösungs-oder Verdünnungsmittels verzichtet wird.

5. Verfahren nach Anspruch 1, dadurch gekennzeichnet, daß der Katalysator ein Alkalisalz einer - schwachen anorganischen Säure, bevorzugt ein Alkalimetallcarbonat, besonders bevorzugt Kaliumcarbonat, ist.

6. Verfahren nach Anspruch 1, dadurch gekennzeichnet, daß der Katalysator in Mengen von 0,1 bis 2,5 Mol-%, bezogen auf Caprolactam, bevorzugt 0,25 bis 1,5 Mol-%, besonders bevorzugt 0,5 bis 1 Mol-%, eingesetzt wird.

7. Verfahren nach Anspruch 3, dadurch gekennzeichnet, daß der Katalysator in heterogener Form vorliegt.

8. Verfahren nach Anspruch 1, dadurch gekennzeichnet, daß man dem Reaktionsansatz nach dem Abkühlen auf eine Temperatur unterhalb des Schmelzpunktes von reinem N-Methylol-caprolactam Impfkristalle aus reinem N-Methylol-caprolactam zusetzt.

9. Verfahren nach Anspruch 8, dadurch gekennzeichnet, daß die Impfkristalle in einer Menge unterhalb von 10 Gew.-%, bezogen auf den Gesamtreaktionsansatz, bevorzugt 0,0001 bis 10 Gew.-%, besonders bevorzugt 0,001 bis 5 Gew.-%, ganz besonders bevorzugt 0,1 bis 1 Gew.-% zugegeben werden.

10. Verfahren nach Anspruch 9, dadurch gekennzeichnet, daß der Zusatz der Impfkristalle bei einer Temperatur von höchstens 65°C, bevorzugt 10 bis 65°C, besonders bevorzugt 20 bis 60°C, ganz besonders bevorzugt 30 bis 55°C, erfolgt.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | CHEMICAL ABSTRACTS, Band 51, Nr. 1, 10. Januar 1960, Spalte 1286e, Columbus, Ohio, US; F.P. SIDEL'KOVSKAYA et al: "N-methylol lactams" & IBID. 901-3 <br> --- | 1-10 | C 07 D 223/10 |
| D,Y | US-A-3 073 843 (S.R. BUC) <br> * Insgesamt * <br> ----- | 1-10 | |

RECHERCHIERTE SACHGEBIETE (Int. Cl.4)

C 07 D 223/00

Der vorliegende Recherchenbericht wurde für alle Patentansprüche erstellt

| Recherchenort | Abschlußdatum der Recherche | Prüfer |
|---|---|---|
| DEN HAAG | 12-04-1988 | ALLARD M.S. |